# EUROPEAN PATENT APPLICATION

(11) **EP 4 578 869 A1**
(43) Date of publication of application: **02.07.2025**
(21) Application number: 23856462.9
(22) Date of filing: 08.08.2023
(51) Int. Cl.: C07K 7/08, C07K 14/00, A61K 38/12, A61K 38/16, A61P 1/00, A61P 5/00, A61P 9/00, A61P 11/00, A61P 19/00, A61P 25/00, A61P 27/02, A61P 27/16, A61P 31/04, A61P 31/12, A61P 37/02

(54) **BRANCHED POLYPEPTIDE VECTOR FOR EFFECTIVELY DELIVERING NUCLEIC ACIDS AND VARIANT THEREOF**

(30) Priority: 23.08.2022 CN 202211014569
(71) Applicant: Suzhou Healirna Biotechnology Co., Ltd., Suzhou, Jiangsu 215000 (CN)
(72) Inventor: LU, Qingqing, Suzhou, Jiangsu 215000 (CN); XIA, Dan, Suzhou, Jiangsu 215000 (CN); YI, Cheng, Suzhou, Jiangsu 215000 (CN); GE, Youzhen, Suzhou, Jiangsu 215000 (CN); HU, Hongpeng, Suzhou, Jiangsu 215000 (CN); JIANG, Jianhao, Suzhou, Jiangsu 215000 (CN); ZHONG, Tianyi, Suzhou, Jiangsu 215000 (CN); ZHANG, Lixia, Suzhou, Jiangsu 215000 (CN); JIA, Kan, Suzhou, Jiangsu 215000 (CN); ZHANG, Yuyang, Suzhou, Jiangsu 215000 (CN)
(74) Representative: Gille Hrabal Partnerschaftsgesellschaft mbB Patentanwälte
(86) International application number: PCT/CN2023/111747
(87) International publication number: WO 2024/041372

(57) **Abstract**

The present invention provides a branched structure peptide carrier and its variations, wherein the branched structure peptide has the following sequence formula:
1. Xaa1(P2)-Xaa1-Xaa1(P1)-Xaa1-Xaa1(P2)
2. Xaa1(P1)-Xaa1-Xaa1(P2)-Xaa1-Xaa1(P2)-Xaa1-Xaa1(P1)
3. Xaa1(P2)-Xaa1-Xaa1(P1)-Xaa1-Xaa1(P1)-Xaa1-Xaa1(P2)
4. Xaa1(P2)-Xaa1-Xaa1(P1)-Xaa1-Xaa1(P2)-Xaa1-Xaa1(P1)-Xaa1-Xaa1( P2)

The technical solution of the present invention enables efficient delivery of nucleic acids to tissues and organs in vivo for targeted therapy. The peptides have several times more conformational flexibility and affinity compared to macromolecular drugs (proteins and antibodies). The branched peptides possess long-lasting in vivo stability while maintaining strong affinity and minimal toxicity. By forming stable nanocomplexes or nanoparticles through electrostatic interactions with nucleic acid molecules, they can facilitate the delivery of nucleic acid drugs and their stable release inside cells, thereby enhancing the activity of nucleic acid-based therapeutics.

## Description

### Field of the Invention

The present invention pertains to the field of nucleic acid delivery, particularly to a branched-chain polypeptide carrier for efficient nucleic acid delivery and its variants.

### Background technology

Nucleic acid-based therapeutics, including plasmid DNA (pDNA), small interfering RNA (siRNA), microRNA (miRNA), antisense oligonucleotides, messenger RNA (mRNA), and aptamers, can alter cellular genetic information, thereby inducing biological effects in cells and organisms. However, the transition of nucleic acids from extracellular to intracellular environments faces challenges such as degradation, precipitation, and protein binding. Polypeptide carriers can protect nucleic acids from degradation, precipitation, and protein interactions, ensuring their functionality within the body.

Nucleic acids typically exhibit a negative charge under physiological conditions. Cationic compounds can electrostatically bind with nucleic acids, compressing and protecting them from enzymatic degradation. These compounds facilitate fusion with cell membranes, enabling nucleic acids to escape from endosomes. Common small-molecule cationic lipids, such as DOTAP (1,2-dioleoyl-3-trimethylammonium-propane) and DDAB (dodecyl-dimethylammonium bromide), achieve moderate transfection efficiency in vitro but fail to meet expectations in vivo. Additionally, cationic polymers like poly-L-lysine, DEAE-dextran, polyethyleneimine (PEI), and chitosan possess a high density of positive charges. Following cellular internalization, their proton sponge effect causes water influx into endosomes, leading to endosomal rupture and the release of nucleic acids into the cytoplasm. However, these polymers often exhibit cytotoxicity and low nucleic acid expression efficiency.

Research into disease-related genetic mechanisms has highlighted nucleic acid-based therapeutics as promising treatments for various diseases. Several nucleic acid drugs have gained approval for clinical use. Nevertheless, rapid degradation, clearance by liver and kidneys, low cellular uptake rates, and poor endosomal escape severely hinder their clinical application. Efficient delivery to target cells is critical for drug efficacy. Polypeptides offer numerous advantages for gene delivery due to their safety, biocompatibility, effectiveness, and ease of synthesis. Derived from endogenous or natural peptides, polypeptides serve roles such as hormones, neurotransmitters, growth factors, and ion channel ligands, with flexible conformations compared to larger biomolecules like proteins and antibodies. For instance, cyclic peptides exhibit prolonged stability in vivo while maintaining high binding affinity and minimal toxicity. Advancements in solid-phase synthesis enable the production of high-purity polypeptides with defined structures and biologically active secondary configurations, such as α-helices and β-sheets. Coupling with sequences or molecules with tissue-targeting properties produces targeted polypeptides. By leveraging non-covalent interactions (e.g., hydrophobic interactions, electrostatic interactions, hydrogen bonding, and π-π stacking), polypeptides can self-assemble into stable nanostructures. These assemblies form stable nanocomplexes or nanoparticles through rational amino acid sequence design, enabling effective nucleic acid delivery and intracellular release to enhance drug activity.

As drug carriers or prodrugs, polypeptides expand their pharmaceutical applications through adsorption, encapsulation, or modification. For example, RGD peptides target integrin receptors for tumor therapy, while combining peptides with antibodies offers novel therapeutic strategies. In antibody-peptide conjugates, antibodies act as targeting entities, and peptides serve as effectors, showing therapeutic potential. Targeting mitochondria in tumor cells, amphipathic α-helical peptides like KLAKLAKKLAKLAK induce apoptosis and exhibit anti-tumor activity. Bee venom peptides, a major component of bee venom, deliver RNA interference (RNAi) therapies to liver cells.

The primary challenge in peptide delivery is degradation. Cyclization reduces proteolytic cleavage, and branched peptides improve half-life, offering promise in cancer targeting. Current strategies to enhance polypeptide stability involve conjugation with carriers (e.g., liposomes or solid nanoparticles) or constructing branched polypeptides with defined and stable nanoscale formulations. Compared to linear peptides, branched polypeptides have precise chemical structures and greater stability, making them highly suitable for therapeutic applications.

### SUMMARY OF THE INVENTION

Based on the above background, the present invention provides a branched structure polypeptide carrier with high gene delivery efficiency and stable structure for effectively delivering nucleic acids, and its variations. The technical solution adopted is as follows:

According to one aspect of the present invention, a branched structure polypeptide is provided with the following sequence formula:
Xaa1(P2)-Xaa1-Xaa1(P1)-Xaa1-Xaa1(P2),
or Xaa1(P1)-Xaa1-Xaa1(P2)-Xaa1-Xaa1(P2)-Xaa1-Xaa1(P1),
or Xaa1(P2)-Xaa1-Xaa1(P1)-Xaa1-Xaa1(P1)-Xaa1-Xaa1(P2),
or Xaa1(P2)-Xaa1-Xaa1(P1)-Xaa1-Xaa1(P2)-Xaa1-Xaa1(P1)-Xaa1-Xaa1(P2);

Where:
P1=(Lys-Xaa2)n-(Lys-Xaa2-Xaa3)m-C1-L, orP1= (Lys-Xaa2)n-(Lys-Xaa2-Xaa3)m-L, or P1 = (Lys-Xaa2)n-(Lys-Xaa2-Xaa3)m; P2 = (Leu-Xaa4)o-(Leu-Xaa4-Xaa5)g-C2, or P2 = (Leu-Xaa4)o-(Leu-Xaa4-Xaa5)g;
**Xaa1** represents Aspartic acid (Asp), Glutamic acid (Glu), or Lysine (Lys);
Xaa2, Xaa3 represent any of Aspartic acid (Asp), Glutamic acid (Glu), Lysine (Lys), Arginine (Arg), Alanine (Ala), Glycine (Gly), Serine (Ser), Asparagine (Asn), or Glutamine (Gln);
Xaa4, Xaa5 represent any of Glycine (Gly), Serine (Ser), Tyrosine (Tyr), Threonine (Thr), Alanine (Ala), Valine (Val), Leucine (Leu), Isoleucine (Ile), Proline (Pro), Phenylalanine (Phe), Tryptophan (Trp), or Methionine (Met);
C1 and C2 are Cysteine (Cys);
L is an optional ligand, which may be present or absent, and can independently be selected from spermidine or putrescine, polylysine, polyarginine, MPG peptide, HIV-binding peptide Tat, SAP peptide, MAP peptide, KALA peptide, FGF peptide, HSV peptide, RGD peptide, GLP-1 peptide, PEP-1 peptide, signal peptides or signal sequences, cytokines, growth factors, hormones, small molecular drugs, carbohydrates, therapeutic active proteins or peptides, antigens or antigen epitopes, antibodies, antibody receptor ligands, synthetic ligands, receptor inhibitors or antagonists, immunostimulatory proteins or peptides, protamine, nucleolar proteins, transferrin, polycationic peptide sequences, polyamines, peptide nucleic acids, nucleic acid embedding agents, nuclear localization signals or sequences (NLS), cell-penetrating peptides, carbohydrates, mannose, galactose, small molecular agonists, poly-L-lysine (PLL), basic peptides, calcitonin peptides, lactoferrin, histones, VP22-derived or similar peptides, or VP22 (herpes simplex) among others;
m, n, o, g may independently be selected from any of the values 0, 1, 2, 3, 4, 5, 6, 7, 8, 9, or 10;
The branched structure polypeptide contains a length of 20 to 150 amino acid residues;
The branched structure polypeptide contains at least one disulfide bond coupling bridge;
The branched structure includes bifurcated, trifurcated, quadrifurcated, or Penta furcated forms.

According to another aspect of the invention, a branched structure composition is provided, wherein the branched structure polypeptide composition is used independently or in combination with one or more of the branched structure polypeptides described in the aforementioned solution.

According to yet another aspect of the invention, a branched structure polypeptide-nucleic acid complex is provided. The branched structure polypeptide-nucleic acid complex is formed by the branched structure polypeptide composition, which further allows for mixing to include or carry one or more nucleic acids to form the branched structure polypeptide-nucleic acid complex.

Further, the nucleic acids mentioned herein include naturally occurring DNA, RNA, or DNA or RNA extracted or synthesized through artificial design; the DNA or RNA extracted or synthesized through artificial design includes small interfering RNA (siRNA), messenger RNA (mRNA), microRNA (miRNA), small activating RNA (saRNA), antisense nucleic acids, or immunostimulatory nucleic acids, etc.

Further, the branched structure polypeptide-nucleic acid complex can effectively carry nucleic acids across animal and human cell membranes and release the nucleic acids.

Further, the branched structure polypeptide-nucleic acid complex can be used in combination with drugs or therapeutic agents. For example, the drugs or agents include peptides, antibodies, enzymes, hormones, antitumor drugs, anti-lipid drugs, antibiotics, anti-inflammatory agents, cytotoxic agents, cell growth inhibitors, immunomodulators, or neuroactive agents.

Further, the branched structure polypeptide-nucleic acid complex contains complexes formed by C12-C18 alkyl or C6-C18 alkyl containing unsaturated bonds with the polypeptide.

Further, the aforementioned branched structure polypeptide composition or complex may contain one or more surfactants, liposomes, lipidoids, alcohol-based lipids, transporters, phospholipids, or any combination of these.

Preferably, the liposomes include, but are not limited to, N-(2,3-dioleoyloxy)propyl)-N,N,N-trimethylammonium chloride (DOTAP), N-(2,3-dioleoyloxy)propyl)-N,N,N-trimethylammonium chloride (DOTMA), distearoylphosphatidylcholine (DSPC), dioleoylphosphatidylcholine (DOPC), dipalmitoylphosphatidylcholine (DPPC), dioleoylphosphatidylglycerol (DOPG), dioleoylphosphatidylethanolamine(DOPE), 3,β-N,(N',N'-dimethylaminoethane)-carbamoyl]cholesterol(DC-chol), dimyristoylphosphatidylcholine (DMPC), dimyristoylphosphatidylethanolamine (DMPE),dioleoyldimethylammonium chloride (DODAC), 1-palmitoyl-2-oleoyl-sn-glycero-3-phosphocholine (POPC), 1,2-dioleoyl-3-trimethylammonium-propane (DOTAP-CI), 1,2-dioleyl-3-dimethylamino-propane (DODMA), 1,2-dimyristoyl-rac-glycerol-3-methoxy polyethylene glycol (DMG-PEG2000), 2-(spermidine amido)ethyl)-N,N-dimethyl-trifluoroacetic acid ammonium (DOSPA), 1,2-dioleoyl-3-dimethylammonium-propane (DODAP), 2,3-dioleoyl-oxy-N-[2-(spermidine carboxy-aminoethyl]-N,N-dimethyl-1-trifluoroacetic-propane (DOSPA), or commercialized liposomes such as DOGS, DOEPC, DPhPE, DPPE, O-Chol, POPE, or DPPG, or any mixture of the above liposomes.

Further, the branched structure polypeptide composition or complex may contain one or more pharmaceutically acceptable carriers, buffers, diluents, excipients, or any combination of these.

Further, the carrier is selected in accordance with the method of administration, in either solid or liquid form. The administration routes include, but are not limited to, intradermal or transdermal, oral, parenteral, including subcutaneous, intramuscular, or intravenous injections, topical or intranasal routes. The dosage of the invention is determined based on animal models. These models include humans and animals, including but not limited to, livestock such as feline or canine subjects, farm animals such as but not limited to cows, horses, goats, sheep, and pigs, research animals such as mice, rats, rabbits, goats, sheep, pigs, dogs, cats, etc., poultry such as chickens, turkeys, songbirds, and humans, goats, cows, pigs, dogs, cats, monkeys, apes, or rodents, including mice, hamsters, and rabbits.

According to yet another aspect of the invention, the branched structure polypeptide composition or complex described in the technical solution is used in the preparation for the prevention, treatment, and/or improvement of diseases selected from the following: cancer or tumor diseases, viral or bacterial infections, genetic diseases, respiratory diseases, cardiovascular diseases, neurological diseases, digestive diseases, bone diseases, connective tissue diseases, immune deficiency diseases, endocrine diseases, eye diseases, and ear diseases.

According to yet another aspect of the invention, the branched structure polypeptide composition or complex described in the technical solution is used for the prevention, treatment, and/or improvement of animal diseases, used in veterinary pharmaceutical compositions, or as an animal vaccine.

The branched structure polypeptide or composition described in the technical solution of the present invention is capable of delivering RNA into animal/human cells, tissues, or individuals in a kit.

Preferably, the branched structure polypeptide described in the present invention is not limited to the following subset of formulas, including one or more combinations of them, with amino acid sequences as shown in Table 1, wherein a disulfide bond is formed between two cysteine (Cys) residues in the sequence:

**Table 1**

| | |
|---|---|
| Sequence 1 | K(LSLLSLC)KKK(LSLLSLC) |
| Sequence 2 | K(LLALLAC)KK (KS) KK(LLALLAC) |
| Sequence 3 | K(LLAC)KK(KD)KK(KD)KK(LLAC) |
| Sequence 4 | K(LLA)KK(KRC)KK(LLA)KK(KRC)KK(LLA) |
| Sequence 5 | K(LAALAALAALAALAAC)KKKK(LAALAALAALAALAAC) |
| Sequence 6 | K(LSALSALSALSAC)KK (KKKKK) KK(LSALSALSALSAC) |
| Sequence 7 | K (LAALAALAAC)KK(KAKAK)KK(KAKAK)KK(LAALAALAAC) |
| Sequence 8 | K(LAALAALAA)KK(KRKRRC)KK(LAALAALAA)KK(KRKRRC)KK(LAALAALAA) |
| Sequence 9 | K(LAALAALAALAALAALAALAAC)KKKK(LAALAALAALAALAALAALAAC) |
| Sequence 10 | K(LAALAALAALAALAALAALAAC)KK (KSKKSKKSKKSKKSK) KK(LAALAALAALAALAALAALAAC) |
| Sequence 11 | K (LAALAALAALAALAAC)KK(KDKDKDKDKDKD)KK(KDKDKDKDKDKD)KK(LAALAALAALAALAAC) |
| Sequence 12 | K(LAALAALAALAALAA)KK(KSKKSKKSKC)KK(LAALAALAALAALAA)KK(KSKKSKKSKC)KK(LAALAALAALAALAA) |

The invention first prepares a branched-chain structure polypeptide via solid-phase synthesis, then mixes it with mRNA and lipid in a two-step process to form a nanoparticle complex, followed by physicochemical property characterization and in vitro and in vivo experimental validation. The main obstacle to peptide delivery in the present invention is the degradation of the peptide. Cyclization can reduce protein cleavage, and branched peptides have the characteristic of improving half-life. The current strategy for solving the stability of peptide drugs is to connect them with carriers (such as liposomes and solid nanoparticles), or to construct multi-branched peptides to form nanodevices with specificity and stability. Branched-chain peptides have a defined chemical structure and are more stable compared to linear peptides. The objective is to provide a structurally stable branched-chain polypeptide carrier for efficient nucleic acid delivery.

The technical solution of the multi-branched-chain polypeptide composition of the present invention provides a multi-branched-chain polypeptide with high gene delivery efficiency, structural stability, and good biocompatibility, along with its variations. The technical solution can efficiently deliver nucleic acids to tissues and organs in vivo to achieve targeted therapy. Compared to existing delivery carriers, the invention offers a series of beneficial technical effects. Peptides have several times the conformational flexibility and affinity of large molecular drugs (proteins and antibodies). Multi-branched peptides have long-term in vivo stability, while maintaining strong affinity and minimal toxicity.

The multi-branched-chain polypeptide described in the invention, as a new generation of biomaterials, possesses superior biological and chemical activity. Therefore, by reasonably designing the amino acid sequence of the polypeptide, stable nano-complexes or nanoparticles can be formed with nucleic acid molecules through electrostatic interactions, enabling the delivery of nucleic acid drugs and their stable release within cells, enhancing the activity of nucleic acid drugs.

The preparation of the branched-chain polypeptide composition in the present invention is relatively controllable, has good biodegradability and safety, low side effects, and enhances the therapeutic index. The composition exhibits good biological response effects and can effectively carry nucleic acid drugs into cells in tissues and organs in vivo, releasing the nucleic acid and generating therapeutic effects through endosomal escape.

The branched-chain polypeptide composition in the present invention effectively enhances the structural robustness, cell membrane permeability, and metabolic stability of the polypeptide molecules by the regular arrangement of the amino acid sequence and the disulfide bond coupling technology. This provides an optimized technical solution for how to safely and efficiently deliver specific or normal nucleic acid sequences into the cells or tissues of patients or animals in molecular medicine therapies.

### BRIEF DESCRIPTION OF THE DRAWINGS

The following drawings further illustrate the present invention, and should not be construed as limiting the scope of the invention.
Figure 1: Liquid chromatography graph of two branched-chain polypeptides.
Figure 2: Mass spectrometry graph of two branched-chain polypeptides.

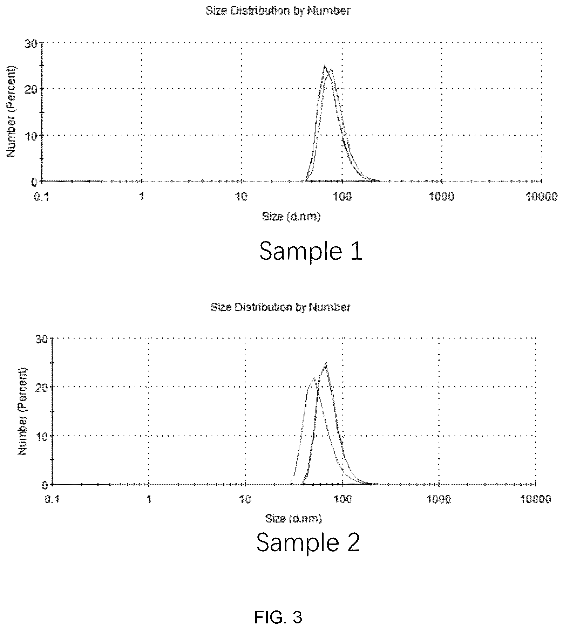

Figure 3: Particle size distribution graph of the branched-chain polypeptide/lipid/mRNA complex formulation.
Figure 4: Gel electrophoresis graph of the branched-chain polypeptide/lipid/mRNA complex formulation.
Figure 5: Schematic diagram of the branched-chain polypeptide/lipid/mRNA complex formulation transfecting 293T cells and expressing the target fluorescent protein.
Figure 6: In vivo delivery of mRNA by the branched-chain polypeptide/lipid/mRNA complex formulation in mice and expression of the target gene protein.
Figure 7: Gel electrophoresis graph of the branched-chain polypeptide and mRNA formulation.
Figure 8: Fluorescent expression image of the branched-chain polypeptide/lipid/mRNA complex formulation transfecting 293T cells.
Figure 9: Toxicity graph of the branched-chain polypeptide/lipid/mRNA complex formulation in 293T cells.
Figure 10: Expression of luciferase in vivo in mice after administration of the branched-chain polypeptide/lipid/mRNA complex formulation.

### DETAILED DESCRIPTION OF THE PREFERRED EMBODIMENTS

### Embodiment 1: Preparation of the Delivery Carrier

### Step 1: Preparation of Peptide Resin

1. Select the starting resin for solid-phase synthesis based on the target sequence, and weigh the corresponding starting resin based on the resin substitution level and the scale of the synthesis. The formula for calculating the weight of the starting resin is as follows:
   The weight of the starting resin (g) = Synthesis scale (mmol) / Resin substitution degree (mmol/g)
2. Place the weighed starting resin into a 100 mL reaction column. Add DCM (dichloromethane) or DMF (N,N-dimethylformamide) at a ratio of 8-10 mL per gram of resin, or a mixture of both solvents. Nitrogen gas is bubbled through for swelling for about 30 minutes. After that, the solution is drained, and the resin is washed three times with DMF.
3. Add DBLK (20% piperidine/DMF mixture) solution to the reactor to remove the Fmoc protecting group. To ensure complete removal, this step is repeated twice, for 5 minutes and 15 minutes respectively.
4. After the Fmoc protection group is removed, the reaction is tested using the ninhydrin color reaction method. A positive result (color development) indicates successful deprotection.
5. After the ninhydrin test, wash the resin with DMF 6 times, each washing for 1-2 minutes.
6. After cleaning, add the amino acids to be coupled in the target peptide sequence along with solid and liquid condensation reagents to carry out the coupling reaction. The reaction time is 1-2 hours (the condensation reagents used in this experiment are HBTU (benzotriazol-1-yl-oxytris(dimethylamino)phosphonium hexafluorophosphate) / DIEA (N,N-diisopropylethylamine), reaction time: 1 hour).
7. After the reaction, a small amount of resin is taken for the ninhydrin color reaction test. A negative result (no color) indicates complete coupling. If a positive result is obtained, it suggests that some free amines are still present, indicating incomplete coupling. In this case, further coupling steps are needed until the reaction is complete.
8. After the reaction is complete, wash the resin three times with DMF and drain the washing solution. Repeat steps 3-7 until the final amino acid of the target peptide sequence is coupled (solid-phase synthesis is generally carried out from the C-terminal to the N-terminal).
9. Once the entire peptide sequence has been coupled, remove the final Fmoc group, shrink the resin with methanol, and obtain the peptide resin (this experiment involves two methanol shrinkage steps, each for approximately 10 minutes).

### Step 2: Preparation of Crude Peptide

1. Weigh the peptide resin from step 1, and add cleavage reagent to cleave the peptide from the resin, removing both the solid-phase resin and the side-chain protecting groups of the amino acids in the sequence. The ratio and cleavage time of the reagent are selected according to the length of the peptide sequence and the complexity of the protecting groups used. (In this experiment, the cleavage reagent ratio is VTFA: phenylmethanethiol: phenylmethanol: EDT = 90:5:3:2, and the cleavage time is 2 hours).
2. After cleavage, filter out the resin and retain the filtrate. Add anhydrous ether to the filtrate at a ratio of 1 mL filtrate to 10 mL ether, and allow the mixture to settle for 20-30 minutes (20 minutes for this experiment).
3. After settling, centrifuge to remove the supernatant and obtain the filter cake. Add anhydrous ether again and repeat the washing and centrifuging process three times to thoroughly clean the filter cake.
4. After washing, place the filter cake in a drying chamber and vacuum dry to obtain crude peptide.

### Step 3: Peptide Cyclization and Final Product Preparation

1. Take the crude peptide from step 2, grind it finely, and dissolve it in an appropriate solvent (water or a mixture of water and acetonitrile) to achieve a clear solution for analysis.
2. According to the requirements of the peptide, if cyclization is not needed, select appropriate gradient elution conditions for purification by preparative chromatography to further purify the sample. If cyclization is required, choose appropriate cyclization reagents (such as hydrogen peroxide or iodine) for cyclization. After cyclization, further purification is performed.
3. After one or more rounds of purification to achieve the desired purity, concentrate the sample by rotary evaporation and then freeze-dry under vacuum.
4. After lyophilization, the final peptide product is obtained. The sample is analyzed by HPLC (high-performance liquid chromatography) and identified by MS (mass spectrometry).
5. Once HPLC purity and molecular weight determination by mass spectrometry are confirmed, the product is stored at -20°C for further experiments.

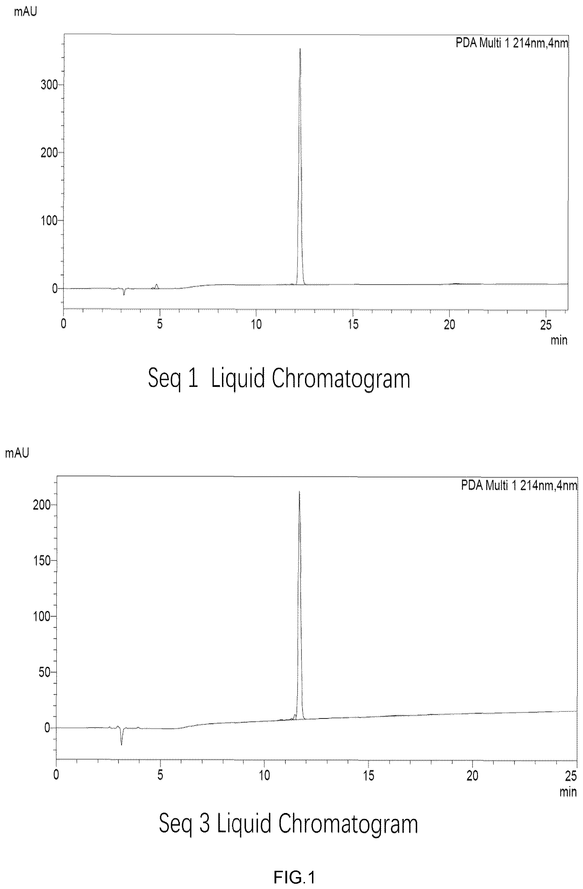

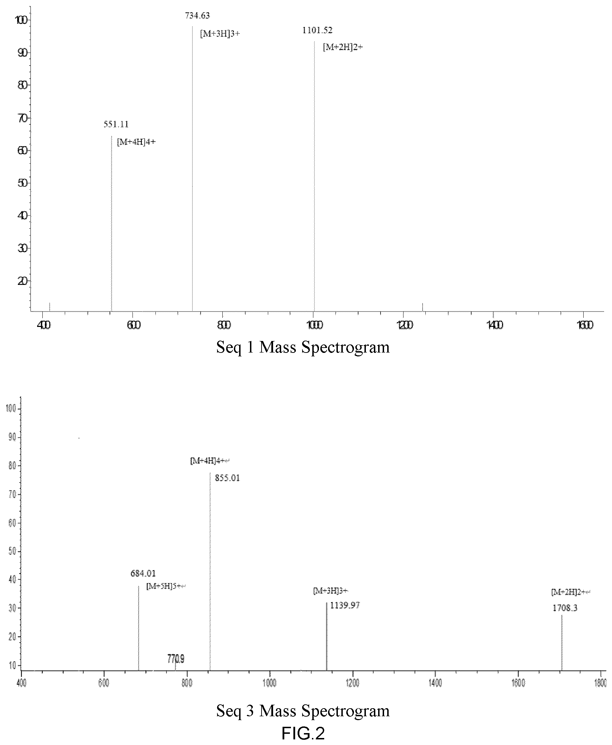

As shown in Figure 1, the purity of Branch Chain Peptide Sequences 1 and 3 are 97% and 95%, respectively. As indicated in Figure 2, the molecular weight matches the theoretical molecular weight, confirming that the synthesized branched-chain peptides meet the experimental requirements.

### Embodiment 2: Preparation Method of Nucleic Acid Delivery Carrier.

First, four kinds of complex formulations of branched-chain peptides (sequences 1-4) with mRNA were prepared. The mass ratio of branched-chain peptide to mRNA ranged from 30:1 to 1:30. Then, cationic lipids/neutral phospholipids/cholesterol/PEG-modified lipids were mixed in a molar ratio of 20-50:5-25:10-50:0.5-15 with the complex formulation from the first step. The mass ratio between the lipid mixture and the peptide/mRNA complex was 1:20 to 20:1. This mixture was further combined using a microfluidic chip to form complex particles. Ethanol was removed by ultrafiltration, and the final lipid/branched-chain peptide/mRNA complex formulation was collected.

Sample 1: Peptide sequence 1 with mRNA at a 1:1 mass ratio, mixed with Dlin-MC3-DMA, DSPC, cholesterol, and PEG-DMG in ethanol at a 1:8 mass ratio.

Sample 2: Peptide sequence 1 with mRNA at a 1:1 mass ratio, mixed with Dlin-MC3-DMA, DSPC, cholesterol, and PEG-DMG in ethanol at a 1:10 mass ratio.

Sample 3: Peptide sequence 1 with mRNA at a 1:1 mass ratio, mixed with Dlin-MC3-DMA, DSPC, cholesterol, and PEG-DMG in ethanol at a 1:12 mass ratio.

Sample 4: Peptide sequence 1 with mRNA at a 1:1 mass ratio, mixed with Dlin-MC3-DMA, DSPC, cholesterol, and PEG-DMG in ethanol at a 1:15 mass ratio.

### Embodiment 3: Particle Size Measurement Experiment

The Malvern Zeta Sizer Nano ZS (Malvern Nano Particle Size Analyzer) was used to measure the particle size (Size) and polydispersity index (PDI) of Samples 1-4 by dynamic light scattering (DLS). The results showed that the lipid/branched-chain peptide/nucleic acid complex particles had a particle size of approximately 100 nm, with PDI around 0.2, indicating that the formulation had well-controlled particle sizes.

**Table 2:**

| Experiment No. | Particle Size (nm) | Polydispersity Index (PDI) |
|---|---|---|
| 1 | 130.3 | 0.138 |
| 2 | 128.6 | 0.296 |
| 3 | 142.9 | 0.211 |
| 4 | 125.3 | 0.195 |

### Embodiment 4: Agarose Gel Electrophoresis Experiment

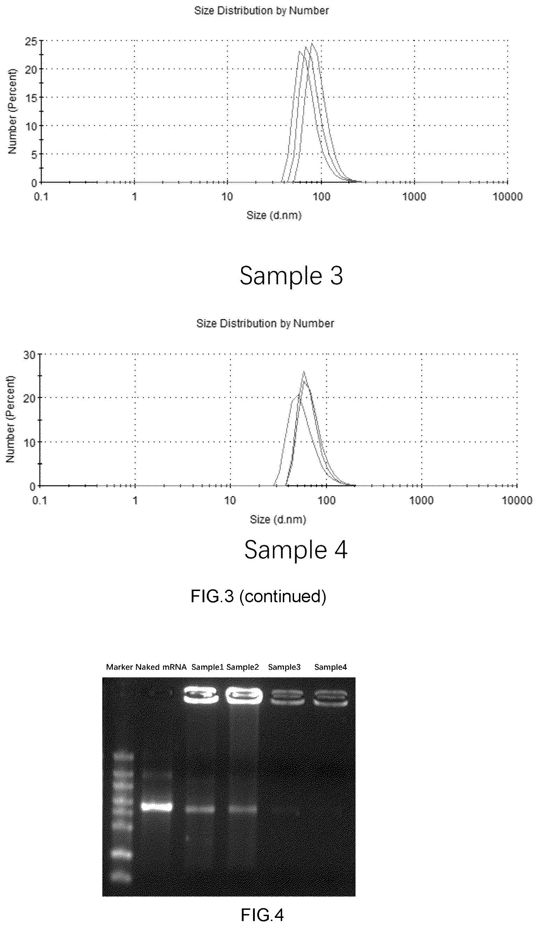

To investigate the binding ability between the branched-chain peptide and the nucleic acid solution, agarose gel electrophoresis was performed.
1. Dissolve 1.5 g of agarose in 150 mL of 1X TBE buffer to prepare a 1% gel.
2. Add RNA fluorescence dye, mix thoroughly, and pour into the gel plate. The lipid/branched-chain peptide/nucleic acid complex formulation was mixed with 2X buffer at a 1:1 ratio and set aside. Add an appropriate amount of TBE solution to the electrophoresis tank.
3. Set the voltage to 120V and run for 20 minutes.
The results showed that when the sample well fluoresced brightly, it indicated that mRNA was completely encapsulated. When no fluorescence was observed, or distinct RNA bands appeared outside the sample well, it indicated that mRNA was not encapsulated. As shown in Figure 4, Samples 3 and 4 could completely encapsulate the nucleic acid.

### Embodiment 5: In Vitro Fluorescent Transfection Experiment.

293T cells were seeded onto 24-well plates with 1 mL of culture medium per well and a cell density of 1×10^5 cells/well. After 12 hours of culture, 1 µg of mRNA formulation was added to 400 µL of serum-free medium and mixed. The positive control group was 3 µL of Lipofectamine 2000 and 1 µg of EGFP added to 400 µL of serum-free medium, mixed, and incubated for 10 minutes. The medium was then supplemented with 600 µL of DMEM complete medium. The negative control group was the naked mRNA group, in which 1 µg of mRNA was added to 1 mL of serum-free medium.

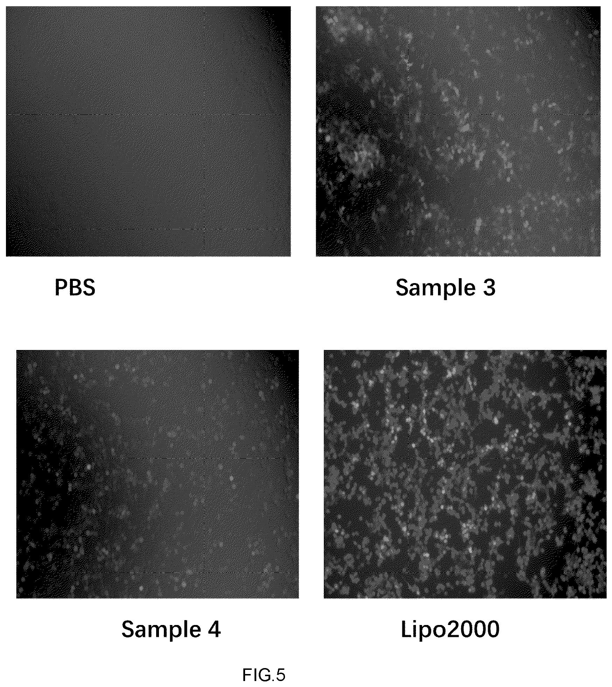

After 20 hours, cells were imaged using a fluorescence microscope. As shown in Figure 5, both the Lipofectamine 2000 group and the nucleic acid delivery carrier group exhibited green fluorescence signals, while the naked mRNA group showed no significant green fluorescence. This indicates that the lipid/branched-chain peptide/nucleic acid complex formulation can effectively deliver mRNA into cells and express the target fluorescent protein.

### Embodiment 6: In Vivo Expression of Target Fluorescent Protein in Mice.

BALB/c female mice aged 6-8 weeks were used in the experiment. The lipid/branched-chain peptide/nucleic acid complex formulation (Sample 3) encapsulating mRNA encoding luciferase was injected intramuscularly at a dose of 100 µL (0.1 µg/µL). Fourteen hours after injection, the luciferase substrate (Luciferin) was injected intraperitoneally, and in vivo imaging of small animals was performed. After the substrate was exhausted, Luciferin was re-injected, and the mice were dissected to observe the spleen, liver, and lung tissues.

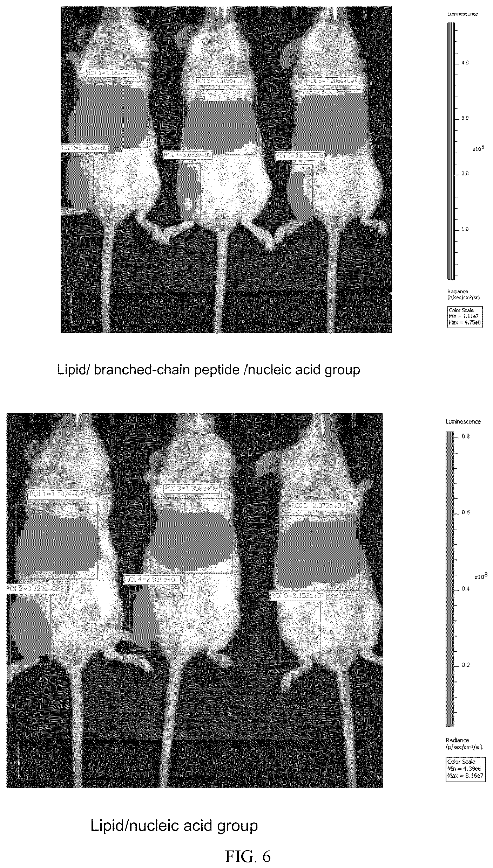

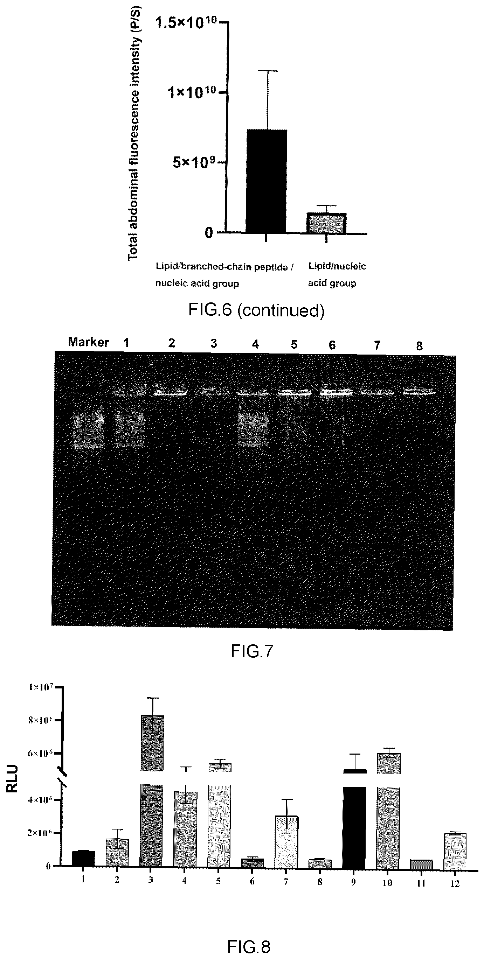

As shown in Figure 6, mice in the lipid/branched-chain peptide/nucleic acid complex formulation group exhibited significant fluorescence in their bodies. Post-mortem examination of the organs revealed that fluorescence was mainly concentrated in the spleen and liver. This demonstrates that the lipid/branched-chain peptide/nucleic acid complex formulation effectively delivers mRNA and expresses the target gene protein in vivo.

### Embodiment 7: T Agarose Gel Electrophoresis Experiment (Further Analysis)

A 1.5% agarose gel was prepared by dissolving agarose in a TBE buffer using a microwave. After adding the sub-green dye, the solution was poured into the gel plate, and a comb was inserted. Peptide samples (sequences 9-12) were mixed with mRNA and loading buffer in a 1:1 ratio and set aside. The gel was loaded with Marker and the samples, then subjected to electrophoresis at 190 V for 20 minutes, and images were captured using a gel imaging system.

As shown in Figure 7, the bands from left to right represent Marker, sequence 9 peptide with mRNA (1:1 and 1:2 ratios), sequence 10 peptide with mRNA (1:1 and 1:2 ratios), sequence 11 peptide with mRNA (1:1 and 1:2 ratios), and sequence 12 peptide with mRNA (1:1 and 1:2 ratios). The results indicate that all the complexes remained within the sample wells, demonstrating that the peptide carriers encapsulated the mRNA, effectively protecting it from degradation.

### Embodiment 8: Preparation of Nucleic Acid Delivery Carrier

Peptides from sequences 5-12 were mixed with mRNA at a 1:1 mass ratio. Dlin-MC3-DMA, DSPC, cholesterol, and PEG-DMG were dissolved in ethanol and mixed with the first-step complex formulation at a 1:12 mass ratio to obtain the nanoformulation. These formulations were named Samples 5-12.

### Embodiment 9: Particle Size Measurement

Using the Malvern Zetasizer NanoZS, the particle size, polydispersity index (PDI), and zeta potential (Zeta P) of the nano-formulations (Samples 5-12) were measured by dynamic light scattering (DLS). The results are shown in Table 3, indicating that the particle sizes of the formulations are around 100 nm, with zeta potentials mostly around 20 mV. The PDI values range from 0.1 to 0.4, indicating good particle uniformity.

**Table 3**

| Sample Number | Particle Size (nm) | Polydispersity Index (PDI) | Zeta Potential (mV) |
|---|---|---|---|
| 5 | 114.11 | 0.31 | 21.6 |
| 6 | 132.43 | 0.33 | 23.53 |
| 7 | 110.90 | 0.17 | 21.73 |
| 8 | 154.17 | 0.22 | -0.06 |
| 9 | 114.23 | 0.26 | 22.57 |
| 10 | 151.23 | 0.41 | 23.10 |
| 11 | 109.20 | 0.27 | 20.03 |
| 12 | 184.23 | 0.22 | 21.67 |

### Embodiment 10: In Vitro Cell Transfection Experiment

293T cells were seeded in 96-well plates at a density of 3×10^4 cells per well and cultured for 24 hours until they adhered. Nanoparticle formulations (Samples 1-12) were prepared according to the method described in Example 8 and added to the cells. After 24 hours of incubation, a luciferase assay was conducted using the Bright-Lite^{™} Luciferase Assay System. Luminescence was measured with a microplate reader. Figure 8 shows that the nanoparticle formulations can successfully deliver mRNA into 293T cells, resulting in fluorescent protein expression, indicating that the branched-chain peptides can effectively carry nucleic acids into cells. The branched-chain peptide lipid nanoparticle complexes demonstrated good expression levels at the cellular level.

### Embodiment 11: Cell Cytotoxicity Experiment

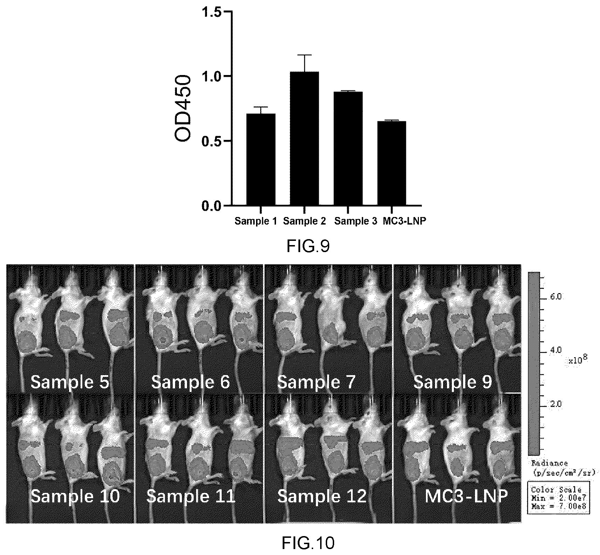

HEK-293T cells were seeded in 96-well plates at a density of 3,000 cells per well and cultured overnight at 37°C and 5% CO2. After 24 hours, the medium was discarded, and different concentrations of drugs were added. MC3-LNP (commercially available) served as the control. Nanoparticle formulations (Samples 1-3) were prepared as described in Example 2 and added to the cells. After another 24 hours of incubation, 10 µL of CCK-8 solution was added to each well, and after 2 hours of incubation at 37°C, the absorbance at 450 nm was measured using a microplate reader to calculate cell viability. The cytotoxicity results are shown in Figure 9. Under these experimental conditions, the branched-chain peptide lipid nanoparticle complexes demonstrated lower cytotoxicity compared to the commercial MC3-LNP, showing better biocompatibility. Cell proliferation was significantly higher, and the OD values for Samples 1, 2, and 3 were 0.71, 1.01, and 0.90, respectively, while the OD value for MC3-LNP was 0.65.

### Embodiment 12: In Vivo Luc Protein Expression Experiment in Mice

6-8-week-old female SPF-grade BALB/c mice (n=3) were used in this experiment, housed in SPF-level animal facilities with a 12/12-hour light/dark cycle, a temperature range of 20-26°C, and humidity between 40-70%. Mice were administered a 100 µL (0.05 µg/µL) dose intramuscularly, with formulations prepared according to Example 8. Six hours after injection, 200 µL of 15 mg/mL D-luciferin potassium salt was administered intraperitoneally, and the mice were placed in a supine position on the imaging platform. After 5 minutes, in vivo imaging was performed. After imaging, D-luciferin potassium salt was re-injected, and after 5 minutes, the mice were euthanized, and organs (heart, liver, spleen, lung, kidney) were collected for organ imaging. As shown in Figure 10, compared to the commercial MC3-LNP lipid formulation, the groups with Samples 5, 6, 7, 9, 10, 11, and 12 exhibited significant fluorescence expression in the mice, indicating that the branched-chain peptide lipid nanoparticle complexes had better expression levels in vivo compared to the commercial lipid formulation.

## Claims

1. A branched-chain polypeptide with the following general formula, **characterized by** having the following sequence formula:
• Xaa1(P2)-Xaa1-Xaa1(P1)-Xaa1-Xaa1(P2),
• or Xaa1(P1)-Xaa1-Xaa1(P2)-Xaa1-Xaa1(P2)-Xaa1-Xaa1(P1),
• or Xaa1(P2)-Xaa1-Xaa1(P1)-Xaa1-Xaa1(P1)-Xaa1-Xaa1(P2),
• orXaa1(P2)-Xaa1-Xaa1(P1)-Xaa1-Xaa1(P2)-Xaa1-Xaa1(P1)-Xaa1-Xaa 1(P2);
• wherein:
•
P1 = (Lys-Xaa2)_n-(Lys-Xaa2-Xaa3)_m-C1-L, or
P1 = (Lys-Xaa2)_n-(Lys-Xaa2-Xaa3)_m-L, or
P1 = (Lys-Xaa2)_n-(Lys-Xaa2-Xaa3)_m;
•
P2 = (Leu-Xaa4)_o-(Leu-Xaa4-Xaa5)_g-C2, or
P2 = (Leu-Xaa4)_o-(Leu-Xaa4-Xaa5)_g;
• Xaa1 is aspartic acid (Asp), glutamic acid (Glu), or lysine (Lys);
• Xaa2, Xaa3 are any of aspartic acid (Asp), glutamic acid (Glu), lysine (Lys), arginine (Arg), alanine (Ala), glycine (Gly), serine (Ser), asparagine (Asn), or glutamine (Gln);
• Xaa4, Xaa5 are any of glycine (Gly), serine (Ser), tyrosine (Tyr), threonine (Thr), alanine (Ala), valine (Val), leucine (Leu), isoleucine (Ile), proline (ⁱPro), phenylalanine (Phe), tryptophan (Trp), or methionine (Met);
• C1 and C2 are cysteine (Cys);
• L is selected from spermidine, spermine, polylysine, polyarginine, MPG peptide, HIV-binding peptide Tat, SAP peptide, MAP peptide, KALA peptide, FGF peptide, HSV peptide, RGD peptide, GLP-1 peptide, PEP-1 peptide, signal peptide or sequence, cytokines, growth factors, hormones, small molecule drugs, carbohydrates, therapeutic proteins or peptides, antigens or epitopes, antibodies, antibody receptor ligands, synthetic ligands, receptor inhibitors or antagonists, immunostimulatory proteins or peptides, protamine, nucleolar proteins, transferrin, polycationic peptide sequences, polyamines, peptide nucleic acids, carboxyspermidine, carboxyspermine, spermidine or spermine analogs, nucleic acid intercalators, nuclear localization signals or sequences (NLS), cell-penetrating peptides, TAT, saccharides, mannose, galactose, small molecule agonists, poly-L-lysine (PLL), basic polypeptides, calcitonin peptide, FGF, lactoferrin, histones, VP22 derivatives or analogous peptides, or VP22 (herpes simplex virus);
• M, n, o, and g are independently selected from any of the values 0, 1, 2, 3, 4, 5, 6, 7, 8, 9, or 10;
• the branched-chain polypeptide contains a length of 20-150 amino acid residues;
• the branched structure includes two-branch, three-branch, four-branch, or five-branch configurations;
• the branched-chain polypeptide includes at least one disulfide bridge for coupling.

2. A branched-chain polypeptide composition, **characterized in that** the composition comprises one or more of the branched-chain polypeptides according to claim 1 for independent use or combined application.

3. A branched-chain polypeptide-nucleic acid complex, **characterized in that** the complex is obtained by mixing one or more branched-chain polypeptide compositions according to claim 2 with one or more nucleic acids to form a composite structure.

4. The branched-chain polypeptide-nucleic acid complex according to claim 3, **characterized in that** the nucleic acid includes naturally occurring DNA, RNA, or artificially designed and synthesized DNA or RNA, such as small interfering RNA (siRNA), messenger RNA (mRNA), microRNA, small activating RNA (saRNA), antisense RNA, or immunostimulatory nucleic acids.

5. The branched-chain polypeptide-nucleic acid complex according to claim 4, **characterized in that** the branched-chain polypeptide-nucleic acid complex can efficiently deliver nucleic acids across cell membranes in animals and humans and release them.

6. The branched-chain polypeptide-nucleic acid complex according to claim 3, **characterized in that** the branched-chain polypeptide-nucleic acid complex can be used in combination with drugs or therapeutic agents selected from peptides, antibodies, enzymes, hormones, antitumor drugs, antilipidemic drugs, antibiotics, anti-inflammatory agents, cytotoxic agents, cell growth inhibitors, immunomodulators, or neuroactive agents.

7. The branched-chain polypeptide-nucleic acid complex according to claim 3, **characterized in that** the branched-chain polypeptide-nucleic acid complex comprises complexes formed with C12-C18 alkyl chains or C6-C18 alkenyl groups.

8. The branched-chain polypeptide composition or complex according to anyone of claims 2-7, **characterized in that** the branched-chain polypeptide-nucleic acid complex comprises one or more combinations with surfactants, liposomes, lipid-like particles, ethosomes, carriers, or phospholipids.

9. The branched-chain polypeptide composition or complex according to anyone of claims 2-7, **characterized in that** the branched-chain polypeptide-nucleic acid complex comprises one or more combinations with pharmaceutically acceptable carriers, buffers, diluents, or excipients.

10. A use of the branched-chain polypeptide composition or complex according to anyone of claims 2-7 for preparing products to prevent, treat, or ameliorate diseases, including cancers or tumors, viral or bacterial infections, genetic diseases, respiratory diseases, cardiovascular diseases, neurological disorders, digestive diseases, skeletal diseases, connective tissue disorders, immune deficiencies, endocrine diseases, ocular diseases, and auditory diseases.

11. A use of the branched-chain polypeptide composition or complex according to anyone of claims 2-7 for preparing products to prevent, treat, or ameliorate animal diseases, veterinary drug formulations, or as animal vaccines.
